# EUROPEAN PATENT APPLICATION

(11) **EP 1 013 290 A1**
(43) Date of publication of application: **28.06.2000**
(21) Application number: 99310215.1
(22) Date of filing: 17.12.1999
(51) Int. Cl.: A61L 15/42

(54) **Multi-layered absorbent composites having one or more apertured transfer layers**

(30) Priority: 17.12.1998 US 112659; 03.12.1999 US 454682
(71) Applicant: FORT JAMES CORPORATION, Deerfield, IL 60015 (US)
(72) Inventor: Schmidt, Bradley Gene, Green Bay, WI 54304 (US); Wergin, Jennifer Nicole, Green Bay, WI 54302 (US); Bouchette, Michael Paul, Sherwood, WI 54169 (US)
(74) Representative: Colmer, Stephen Gary

(57) **Abstract**

A multilayer absorbent composite is disclosed. This composite comprises a cover sheet, one or more transfer layers, one or more absorbent core layers, and a barrier back layer. The transfer layer or layers have Z-direction apertures penetrating through the entire layer. The aperture frequency is about 5 to 50 apertures per square inch and each aperture has a diameter of about 0.015 to 0.10 inches. These composites are used as feminine hygiene pads and related applications.

## Description

### BACKGROUND OF THE INVENTION

This invention relates to multilayered composites, comprising a cover sheet, one or more apertured transfer layers, one or more absorbent core layers and a barrier back layer. The composites of this invention have better absorbent properties than prior art products.

### DESCRIPTION OF BACKGROUND ART

Prior art has not taken advantage that apertured transfer layers containing superabsorbents prevent the backward flow of the fluids that are finally absorbed by the absorbent layer. We have discovered that if the apertures are kept within controlled diameter and a controlled frequency per square inch superior absorbent components are obtained.

Representative prior art includes Barnes et al U.S. Patent 5,137,620; McCormack et al U.S. Patent 5,328,759; Lemay et al U.S. Patent 5,374,260; Couteur-Dorschner U.S. Patent 5,401,267; Everhardt et al U.S. Patent 5,801,107; Gilman U.S. Patent 5,803,920 and Menard et al U.S. Patent 5,810,797. For absorbent articles like diapers, feminine hygiene, and the like, prior art has developed many variations of multi-layer structures. Depending upon the specific end-use requirements, these structures usually consist of at least a top sheet or cover sheet, a transfer layer, an absorbent core, and a back sheet. The function of the top sheet or cover sheet provides a comfortable and non-irritating contact with the skin of the user. The function of the transfer layer is to rapidly move the liquid insult away from the top sheet and into the absorbent core. The function of the absorbent core is to provide the liquid holding reservoir of the product. And the function of the back sheet is to provide an impervious barrier to liquid leakage.

Generally speaking, the laws of capillarity suggest that the transfer layer should be relatively dense, in order to provide good and fast wicking. But, as the density of the transfer layer increases, its ability to hold liquid decreases, and thus there is a constant conflict between how fast the transfer layer can move liquid, and how much liquid it can move at any given time.

Once the liquid reaches the absorbent core, the function of the core is to keep it there. To do this efficiently, the absorbent core must almost operate on the reverse principle of the transfer layer. That is, it must have a low density with large pores. The large pores provide the capacity to hold the liquid. Unfortunately, the larger the pore size, the less ability of the absorbent core to retain the liquid under any kind of pressure.

Typical product efficacy measurements for absorbent core products include the rate of liquid transfer, sometimes identified as "strikethrough" in the industry, the capacity of liquid holding, and the ability of the product to retain the liquid under load, sometimes identified as "rewet" in the industry. The smaller the strikethrough time, the better the product performance, and the tower the rewet values, the better the product performance.

To attempt to solve the deficiencies of the transfer layer, which must rapidly move the entire liquid insult away from the top sheet as quickly as possible, one of the prior art techniques utilized to enhance rapid liquid movement has been to punch small holes in the transfer layer. This is exemplified in McCormack et al (5,328,759) in which hydraulic needling (otherwise known as hydroentaglement) of a nonwoven web was used to enhance its liquid distribution properties. This, in effect, creates macro liquid flow channels in the transfer layer, and also provides an increase in holding capacity. The size of the holes are such that a large volume of liquid can rapidly pass through them. The problem with this approach is that the holes can also just as easily allow liquid to pass back out from the absorbent core to the top sheet.

McCormack also teaches the addition of a superabsorbent to one surface of the hydraulically entangled web while in its wet state after hydroentangling. The holes in McCormack's disclosure do serve to facilitate rapid liquid movement and transfer, but they also serve as repositories for the particles of superabsorbent which are added to the web in its wetted stage after the hydraulic needling. As the particles fill the holes, they will necessarily physically plug them, preventing the full enjoyment of their use. Furthermore, McCormack teaches addition of the superabsorbent particles or powder to the web when it is in its wetted condition, following the hydroentaglement process used to generate the holes. This is done to provide some minimal bonding of the superabsorbent to the web so that it does not fall from the surface of web during enjoyment of their use. Furthermore, McCormack teaches addition of the superabsorbent particles or powder to the web when it is in its wetted condition, following the hydroentanglement process used to generate the holes. This is done to provide some minimal bonding of the superabsorbent to the web so that it does not fall from the surface of web during converting operations, usage, and the like. The easy and convenient retention of superabsorbents is also a common deficiency of webs and/or products, and although McCormack's approach does improve retention of the superabsorbents, it is still only a minimal improvement, the thus the success of McCormack's approach has been limited. The prior art has incorporated superabsorbents, in the form of particles, powders, fibers or binders in the absorbent cores to increase holding capacity. Again, McCormack et al is an example. These superabsorbent materials swell and increase the ability of the core to hold liquid quite dramatically. But they suffer from the deficiency of requiring a finite amount of time to swell and become effective, during such time the liquid is still unbound, and the unbound liquid may thus cause rewet or strike-through back to the transfer layer and the top sheet. Everhart et al (5,801,107) teaches that superabsorbents have a tendency to reduce the liquid distribution properties of a web or laminate. This suggests that the liquid insult in a superabsorbent core does not have the opportunity to spread out and utilize the full benefit of the area and mass of the absorbent core. Barnes et al (5,137,600) further teaches that loose fibers, which are often the prime component of the absorbent core, actually reduce the rate of wicking and absorption.

### SUMMARY OF THE INVENTION

This invention relates to multilayered absorbent composite comprising a cover sheet, one or more apertured transfer layers, one or more absorbent core layers and a barrier back layer. The transfer layer or layers have z-direction apertures through the entire layer. The aperture frequency is from about 5 to about 10 apertures for each square inch and each aperture has a diameter of about 0.015 to 0.10 inches. The apertures in the transfer layer or layers are made by flat bed needle punching, perf roll punching, needle roll punching, hydroentangling or aperturing. In the preferred embodiment the aperture frequency is about 9 to 14 apertures per square inch and the diameter of the apertures is controlled to be about 0.037 to about 0.070 inches.

The transfer layer is composed of cellulosic fibers derived from wood pulp; it is also suitable that the transfer layer comprises fused wood pulp or cellulosic fibers, natural fibers, synthetic fibers and mixtures of these. The synthetic fibers in the transfer layer are selected from the group consisting of regenerated cellulose, polyolefins, polyethylenes, polypropylene, polyesters, polyimides, polyamides, inorganic polymers, silica polymers and carbon fibers. The transfer layers and the core layers comprise about 5 to 60 weight percent of a super absorbent composition obtained by treating a crosslinker with an organic polymer or its salt. Suitably the fibers in the transfer layer or layers are bonded together by latex bonding, bicomponent fiber bonding, monocomponent thermoplastic bonding, or thermosetting bonding fibers. The amount of bonding agent incorporated in the transfer layer is controlled to be in the range of about 5 to 30 weight percent, preferably 9 to 25 weight percent. Care is taken to keep the final basis weight of the transfer layer or layers in the range of about 80 to 200 grams per square meter, preferably 100 to 150 grams per square meter. Usually the composite comprises air laid tissue layers however it is advantageous to incorporate a wet laid tissue layer as part of the transfer layer structure. The basis weight of the wet laid paper product is about 10 to 25 grams per square meter, preferably 13 to 20 grams per square meter.

The nonwoven web is formed by use of the following processes: (1) airlaid, (2) carding, (3) melt blown, (4) spunbond, and (5) coforming. The paper web is formed by foudrinier, roof formers, gap formers, cylinder forming or rotoforming papermaking processes.

### BRIEF DESCRIPTION OF THE DRAWINGS

Figure 1 illustrates a specific embodiment of the multilayer composite structure of this invention.
Figure 2 illustrates the liquid multilayer movement through the apertured composite of this invention.
Figure 3 illustrates an airlaid machine, incorporating the use of a carrier web on the foraminous forming wire to retain the superabsorbent material and the process for the manufacture of the multilayer apertured composite of this invention.
Figure 4 illustrates a needle felting process.
Figure 5 illustrates the aperturing of the web using a needle roll.
Figure 6 illustrates the aperturing of the web using a perf roll.

### DETAILED DESCRIPTION OF THE INVENTION

There is strong need for an improved composite product with superior transfer properties and absorbent core properties. Surprisingly, we have discovered an improved multilayer composite by the incorporation of both apertures and superabsorbent in the transfer layers. As taught by Everhardt, et al. U.S. Patent 5,801,107 one would normally expect that the incorporation of superabsorbents in the transfer layer would slow down the transfer of the liquid to the absorbent core. However, what we have discovered is that while the use of apertures in the transfer layer does indeed facilitate the rapid movement of liquid through the transfer layer to the absorbent core, their combination with the use of superabsorbents in the transfer layer of our invention create a superior multilayer composite product. Unlike the teachings of McCormack et al, U.S. Patent 5,328,759 in which a superabsorbent is added to one surface of the web, we have found that an essentially homogenous distribution of superabsorbent within the fibrous structure results in superior wicking and liquid distribution. It might seem at first that the superior performance of our invention disagrees with the teachings of Everhardt, but upon studying the mechanisms involved, we have concluded that the superabsorbents in our transfer layer act just the same as they do in the absorbent core layer in that they require a finite amount of time before swelling and becoming active. But in our invention they do not inhibit the initial passage of liquid through the transfer layer because the insult liquid is rapidly transferred by the apertures, and the product still performs well. Furthermore, we have found that by adding the superabsorbents into the web structure, and retaining them with both a tissue layer and latex bonding, we have largely eliminated the loss of superabsorbent material in subsequent converting operations. We have found that the use of latex bonding not only serves to hold the fibers together and to hold the superabsorbent material in the fibrous matrix, but that the latex bonding in the transfer layer of our composite also serves to deliberately retard the timing of activation of superabsorbent material when wetted so that the liquid insult can more readily pass through our transfer layer without seriously activating the superabsorbent material.

McCormack, Everhardt, and Barnes all teach wet hydraulic needling as the preferred method to generate apertures. McCormack and Everhart both teach that the hydraulic needling of the web is then followed by the addition of superabsorbent materials. In our invention we have created a superior product and process in that the superabsorbent is added to the web while it is in the dry stage, without the need for water as a bonding agent, and in our invention the apertures are made after the web is completely formed with all components, including the superabsorbents. Thus, in our invention, when the apertures are created, there is no adverse tendency for the superabsorbent materials to preferentially fill these apertures.

The superiority of our invention to the prior art products is readily seen and explained by the fact that the unbound liquid in the absorbent core, unbound either from an inadequate reaction time with the superabsorbent, or unbound due to the pressures applied to the product in use (sitting on it, for example) then begins to also react with the superabsorbents in the transfer layer as the unbound liquid attempts to pass back to the transfer layer. But, as these superabsorbents in the transfer layer finally now begin to swell, they basically block the apertures in the transfer layer and prevent liquid strike-through back to the top sheet. Thus, in our invention, the use of apertures and superabsorbents in the transfer layer facilitates rapid and voluminous liquid transfer away from the top sheet and to the absorbent core. Furthermore, our multilayer composite provides superior liquid retention in the absorbent core by subsequent blocking of the apertures in the transfer layer thereby preventing backflow of the liquid insult. In our invention, we clearly show improved liquid holding capacity and improved rewet values over prior art products.

A superabsorbent fibrous web formed from wood pulp or cellulosic fibers, natural fibers, synthetic fibers, or any mixture thereof wherein the web is treated with superabsorbent polymers which produce a multilayered composite having substantially increased absorbency rates and absorbent capacity are disclosed.

The superabsorbent polymer is selected from polyacrylate polymers and their sodium, lithium, or potassium salts. A suitable polyelectrolyte has a molecular weight of at least 150,000, advantageously 190,000 to 220,000.
The alkali-soluble polyacrylates known as polyelectrolytes useful to form the crosslinkable solutions utilized in carrying out this invention can be made by utilizing processes such as emulsion, suspension, bulk or solution polymerization techniques, provided they consist of about 50 to 92 percent by weight of an alkyl acrylate with 1-10m carbon atoms in the alkyl moiety or an alkyl methacrylate with 4-10 carbon atoms in the alkyl moiety, about 8 to about 50 percent by weight of an omega hydroxyl alkyl acrylate having 1-4 carbons in the alkyl moiety. It is preferred to use alkali-soluble lattices having about 15 to about 60 weight percent of non-volatile polymer solids as is set forth below.

Examples of useful alkyl acrylates are methyl acrylate, ethyl acrylate, propyl acrylate, hexyl acrylate and the like. Examples of useful alkyl methacrylates are butyl methacrylates, hexyl methacrylates, octyl methacrylate, decyl methacrylate and the like

Examples of useful omega hydroxy alkyl acrylates are 2-hydroxyethyl acrylate, hydroxymethyl acrylate, 3-hydroxy-propyl acrylate and 4-hydroxybutyl acrylate.

The foregoing polyacrylates are then dissolved in an aqueous alkali metal hydroxide solution. Generally the equivalents of hydroxide solution used are from about 30 to about 70% based on the molar concentration of polymerized monomer and the preferred amount is from about 40 to about 55%. In any event, the amount of hydroxide solution added is sufficient to convert or saponify some of the acrylate esters to alkali metal carboxylates and to neutralize the carboxylic groups of the polyacrylate used into alkali metal carboxylates so that the converted polyacrylate has about 30 to about 70 weight percent of alkali metal carboxylates.

This solution is then rendered crosslinkable by adding about 0.1 to about 10 weight percent based on the dissolved polymer of a water soluble crosslinking agent.

Suitable crosslinking agents are zirconium compounds having a valence of plus four. Suitable zirconium compounds include ammonium zirconium carbonate, zirconium acetylacetonate, zirconium acetate, zirconium carbonate, zirconium sulfate, zirconium phosphate, potassium zirconium carbonate, and zirconium sodium phosphate and sodium zirconium tartrate and mixtures thereof.

Other suitable soluble crosslinking agents are polyhaloalkanols such as 1,3-dichloroisopropanol, 1,3-dibromoisopropanol; sulfonium zwitterions such as the tetrahydrothiophene adduct of novolac resins; haloepoxyalkanes such as epichlorohydrin, epibromohydrin, 2-methyl epichlorohydrin and epidohydrin; polyglycidyl ethers such as glycerine diglycidyl ether, ethylene glycol diglycidyl ether, propylene glycol diglycidyl ether, diethylene glycol diglycidyl ether; and the mixtures of the foregoing.

The cellulosic fibers can suitably be substituted with synthetic fibers and/or natural fibers. The substitution can range from 10 to 50 percent. In some instances the cellulosic fiber is completely replaced with the synthetic fiber and/or natural fibers. Suitably synthetic fibers are selected from regenerated cellulose, polyolefins, polyethylenes, polypropylenes, polyesters, polyamines, polyamides, inorganic polymers, silicon polymers, and carbon fibers. Suitable natural fibers are selected from wool, camel hair, mohair, and other non-cellulosic animal, vegetable, or mineral sources.

Papermaking fibers used to form the paper products of the present invention include cellulosic fibers commonly referred to as wood pulp fibers, liberated in the pulping process from softwood (Gymnosperms or coniferous trees) and hardwoods (angiosperms or deciduous trees). Cellulosic fibers from diverse material origins may be used to form the web of the present invention including non-woody fibers liberated from sugar cane, bagasse, sabai grass, rice straw, banana leaves, paper mulberry (i.e., bast fiber), abaca leaves, pineapple leaves, esparto grass leaves, and fibers from the genus Hesperaloe in the family Agavaceae. Also recycled fibers which may contain any of the above fiber sources in different percentages can be used in the present invention. Suitable fibers are disclosed in U.S. Patent Nos. 5,320,710 and 3,620,911, both of which are incorporated herein by reference.

Papermaking fibers can be liberated from their source material by any one of a number of chemical pulping processes familiar to one experienced in the art including sulfate, sulfite, polysulfite, soda pulping, etc. The pulp can be bleached if desired by chemical means including the use of chlorine, chlorine dioxide, oxygen, etc. Furthermore, papermaking fibers can be liberated from source material by any one of a number of mechanical/chemical pulping processes familiar to anyone experienced in the art including mechanical pulping, thermomechanical pulping, and chemi thermomechanical pulping. These mechanical pulps can be bleached, if one wishes, by a number of familiar bleaching schemes including alkaline peroxide and ozone bleaching. The type of furnish is less critical than is the case for prior art products. In our process coarse hardwoods and softwoods and significant amounts of recycled fiber can be utilized to create useful products.

The latex binder has a glass transition temperature of about -30°C to about +30°C, preferably from -10°C to +10°C. Nitrocellulose, lacquer, styrene acrylic polymers and terpolymer emulsions of vinyl chloride, ethylene and vinyl acetate having a glass transition temperature of about 0° to 3°C are suitable. In general, the latex polymeric binder is chosen from the group consisting of polymers of ethylenically unsaturated monomers, copolymers of ethylenically unsaturated monomers, polymers, and copolymers of conjugated dienes, saturated and unsaturated polyesters, polycarbonates, polyethers, polyurethanes, epoxies, ureaformaldehydes, and phenolformaldehydes. Advantageously, the latex polymeric binder is chosen from the group consisting of copolymers of ethylenically unsaturated monomers such as copolymers of ethylene and vinyl acetate, ethylene and propylene, ethylene and styrene, and polyvinyl acetate, styrene and maleic anhydride, styrene and methyl methacrylate, styrene and ethyl acrylate, styrene and acrylonitrile, methyl methacrylate and ethyl acrylate, methyl methacrylate and acrylonitrile, and vinyl acetate and acrylamide.

The latex binder is selected from the group consisting of aliphatic acrylate acrylonitrile styrene copolymers, n-butyl acrylate acrylonitrile styrene copolymer, n-amyl acrylate acrylonitrile styrene copolymer, n-propyl acrylate acrylonitrile styrene copolymer, n-ethyl acrylate acrylonitrile styrene copolymer, aliphatic acrylate styrene copolymers, n-butyl acrylate styrene copolymer, n-amyl acrylate styrene copolymer, n-propyl acrylate styrene copolymer, n-ethyl acrylate styrene copolymer, starch latex copolymers, animal glue, gelatin, methyl cellulose, carboxymethylcellulose, polyvinyl alcohol, ethylene-vinyl acetate copolymer, vinyl acetate-acrylic copolymer, styrene-butadiene copolymer, ethylene-vinyl chloride copolymer, vinyl acetate polymer, vinyl acetate-ethylene copolymer, acrylic copolymer, styrene-acrylic copolymer, stearylated melamine, hydrophilic epoxy esters, and mixtures of these.

The zirconium crosslinker compounds are selected from the group consisting of ammonium zirconium carbonate, zirconium acetylacetonate, zirconium acetate, zirconium carbonates, zirconium sulfate, zirconium phosphate, potassium zirconium carbonate, zirconium sodium phosphate, sodium zirconium tartrate, and mixtures of these.

Other suitable crosslinkers include 1,3-dichloroisopropanol, 1-3-dibromoisopropanol; tetrahydrothiophene adduct of novolac resins; epichlorohydrin, epibromohydrin, 2-methyl epichlorohydrin and glycerine diglycidyl ether, ethylene glycol diglycidyl ether, propylene glycol diglycidyl ether, diethylene glycol diglycidyl ether; and the mixtures of the foregoing.

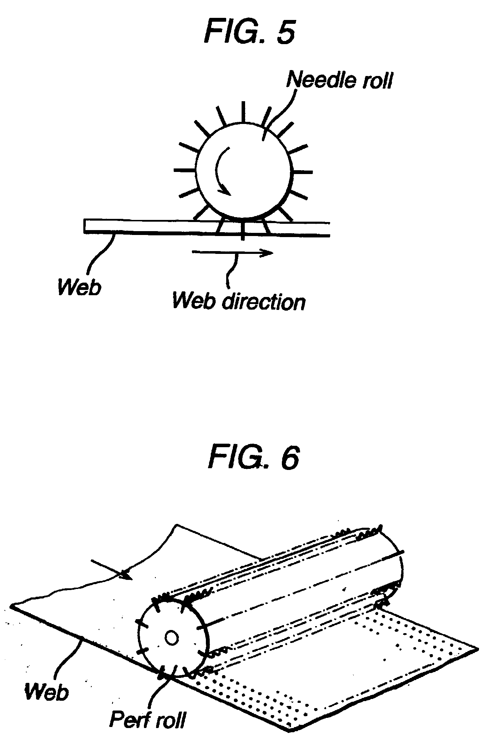

This invention relates to a multilayered absorbent composite comprising a cover sheet, one or more apertured transfer layers, one or more absorbent core layers and a barrier back layer. The transfer layer or layers have Z-direction apertures through the entire layer. The aperture frequency is from about 5 to about 10 apertures for each square inch and each aperture has a diameter of about 0.0125 to 0.10 inches. The apertures in the transfer layer or layers are made by flat bed needle punching, perf roll punching, needle roll punching, hydroentangling or aperturing. In the preferred embodiment the aperture frequency is about 9 to 14 apertures per square inch. The diameter of the aperture is controlled to be about 0.037 to about 0.70 inches. Needle punching is illustrated in Figure 5 and perf roll punching is illustrated in Figure 6.

The transfer layer is composed of cellulosic fibers derived from wood pulp but natural fibers, synthetic fiber and mixtures of these are suitable. The synthetic fibers in the transfer layer are selected from the group consisting of regenerated cellulose, polyolefins, polyethylenes, polypropylenes, polyesters, polyimides, polyamides, inorganic polymers, silica polymers and carbon fibers. The transfer layers and the core layers comprise about 5 to 60 weight percent of a superabsorbent composition obtained by treating the aforementioned crosslinkers with an organic polymer or its salt described hereinabove. The superabsorbent composition comprises about 20 to 50 weight percent of the final composite product. Suitably the fibers in the transfer layer or layers are bonded together by latex bonding, bicomponent fiber bonding, monocomponent thermoplastic bonding, or thermosetting bonding fibers. The amount of bonding agent incorporated in the transfer layer is controlled to be in the range of about 5 to 30 weight percent, preferably 9 to 25 weight percent. Care is taken to keep the final basis weight in the range of about 80 to 200 grams per square meter preferably 100 to 150 grams per square meter. Usually the composite comprises air laid tissue layers. However it is sometimes advantageous to incorporate a wet laid tissue layer as part of the transfer layer structure. The basis weight of the wet laid paper product is about 10 to 25 grams per square meter, preferably 13 to 20 grams per square meter.

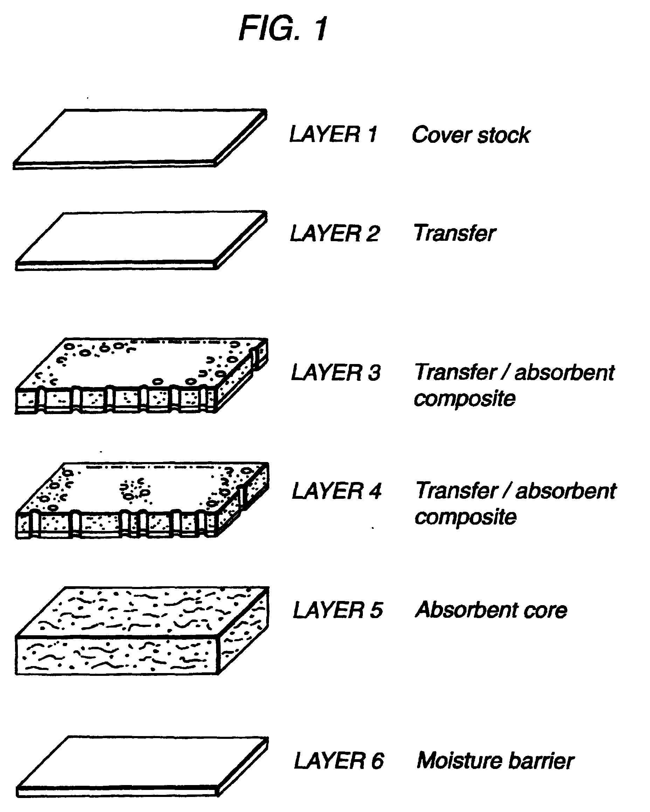

Figure 1 shows a typical multi-layer composite structure, utilizing the benefits of our invention. The cover stock layer (layer 1) may be made from any number of substrates, including carded webs of synthetic fibers, apertured plastic webs, and the like. The purpose of the cover stock is to provide a non-irritating surface of the product to be worn next to the body, and to transfer liquid away from the insult site into the composition of the transfer layer or layers is optional. In Figure 1 we have simply shown an optional conventional densified layer (layer 2) as the first of three transfer layers. Again, the function of a transfer layer is to move liquid away from the cover sheet and toward the absorbent core. The ultimate design, composition, and number of transfer layers will be dictated by the specific product performance requirements.

Layers 3 and 4 in Figure 1 represent the utilization of our invention both in the layers themselves, and in the final composite structure. Again, the number of layers is determined by the final product performance needs, and may readily be adjusted by those skilled in the art. Layers 3 & 4 are essentially the same in Figure 1. Both are constructed through the use of an air laid forming device, and utilize a wood pulp or other carrier web as one part of the transfer layer composite, and an air laid web mixed together with superabsorbents as the other, wherein the two webs are then bonded together with latex bonding, and are then subjected to the aperture creating process. The function of the carrier layer is to act as a holding layer for the superabsorbents as they are added with the wood pulp on top of the tissue. The carrier web not only retains the superabsorbent, but it also prevents the superabsorbent from striking through to the foraminous wire used in the formation of the air laid web and thereby plugging it. In Figure 1, both of the carrier layers are shown at the bottom of the transfer layer, but we believe that in the final product composite they can be used advantageously in either the bottom or the top positions. Carrier layers also are dense when compared to the air laid portion of their composite layer, and this density facilitates increased wicking.

Layer 5 in Figure 1 represents a typical absorbent core. In this case, the core is comprised of unbonded fluff pulp. Layer 5 may also be a bonded air laid wood pulp substrate. Layer 5 in Figure 1 is shown with superabsorbents, but the absorbent core may also be made without the use of superabsorbents. And Layer 6 in Figure 1 is an impervious back sheet, designed to prevent liquid leakage.

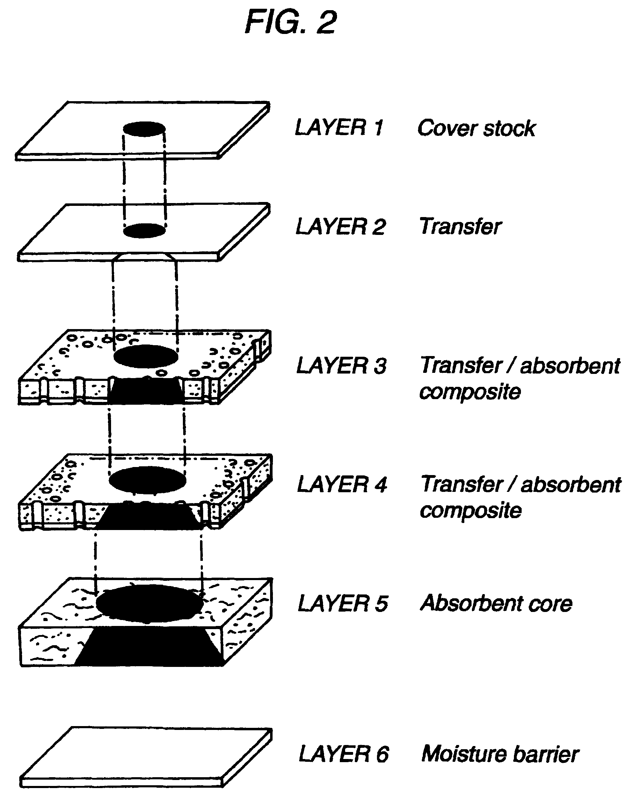

Figure 2 has been drawn to represent an exaggerated view of the liquid movement through a composite structure. As the liquid passes through layer 1, the cover sheet, it passes through essentially with little horizontal or planar wicking. Again, the horizontal or planar wicking of the cover sheet may be adjusted by the proper design of the fibrous structure, the fibers themselves, and selective surface chemistry alteration techniques. Even when the cover sheet is a plastic material, and is not fibrous, it is still possible to affect its ability to be wetted through chemical treatments. However, the most important function of the cover sheet it to move the liquid away rapidly. That is, the area of the liquid insult on the top of the cover sheet must be carried essentially through the sheet into the transfer layer or layers. In transfer layer 2, which is optionally shown as a simple but dense carrier type of fibrous web, (like a wet laid tissue or highly compacted air laid web, for examples) there is a slight horizontal wicking which occurs as the liquid moves from top to bottom. This is advantageous in that it causes the liquid to move away from the insult site faster than it could if it only passed straight through the web. This horizontal wicking is due to the increased fiber density of layer 2. In layer 3, the liquid also exhibits some horizontal wicking in the air laid portion of the composite, but less than in layer 2 due to the decreased density of the air laid portion of the composite. When the liquid hits the carrier web portion of the composite, horizontal wicking occurs slightly more. But the majority of the liquid passed through layer 3 without staying behind due to the vertical holes of our invention which serve to act as conduit transfer tubes. Exactly the same phenomena occurs in layer 4, and for the same reasons.

However, when the liquid hits layer 5, the absorbent core layer, other things begin to happen. First of all, the density of the unbonded fluff of layer 5 is such that horizontal wicking and liquid transfer do not occur to any great extent when the product is not under any pressure or vertical compression.

Basically the same phenomena occurs when the absorbent core is comprised of a bonded air laid substrate. There is little cause to move the liquid, and it simply flows to the point where it meets a superabsorbent material, and then begins to swell the superabsorbent material. But the superiority of our invention utilizes the fact that the product, as used, is most often subjected to some type of vertical pressure as the user is more often walking or sitting than completely standing still. When these vertical pressures are applied the web is densified easily and the increase in density is rapid and sufficient to then cause good horizontal wicking. In layer 5, the liquid insult area is shown as the largest of all within the composite. This large insult area is essential to effect the advantages of our invention in that the more the liquid is spread out horizontally, the less liquid there is at any one spot to try to move back up vertically. When superabsorbents are used in a fluff pulp layer 5 they are also free to act nearly instantaneously in that they are free within the fibrous mass, and are not bonded in any manner. When superabsorbents are used in a bonded air laid substrate for layer 5, they are somewhat less free within the fibrous structure, so their reaction time is somewhat delayed. This delay for bonded absorbent core substrates is nonetheless offset in unbonded fluff pulp absorbent cores in that the density of the bonded substrates is usually slightly higher than the unbonded fluff pulp, and an increase in density facilitates liquid wicking and spreading.

Even though the pressure of use increases the benefit and performance of layer 5 through horizontal or planar wicking, any pressure still naturally causes a tendency for the liquid to move in any free direction, including back up vertically to the transfer layer. This is a deficiency of most absorbent core products, especially those utilizing only vertical holes for rapid liquid transfer. The conduit transfer tubes can just as easily move liquid back up through the tube as they move it down through the tube. But the superiority of our invention lies in that the transfer layer, which contains the conduit tubes also contains superabsorbent material which has been slightly bonded in place during the web forming and bonding operation, and that the apertures in the transfer layer of our invention were created after the web was completely formed and bonded. By utilizing superabsorbents in our transfer layer that were slightly bonded, we have delayed their reaction time when exposed to liquid. That is to say, when liquid hits a superabsorbent material in the transfer layer, the latex coating on the superabsorbent inhibits the initial ability of the liquid to wet the superabsorbent and the subsequent ability of the superabsorbent to swell to its fullest. The net effect is a time delay before the superabsorbent in the transfer layer of our invention can act. This time delay is advantageous in that it then allows the liquid to pass freely through the apertures of our transfer layer to the absorbent core. We have found that the time required to allow the insult liquid to pass through the transfer layer into the absorbent core and begin wicking and retention in the absorbent core is enough so as to accommodate the built-in reaction delay of the superabsorbent action in the transfer layer. Thus, as the vertical pressures are applied to the product during use, and the liquid in the absorbent core attempts to come back into the transfer layer, the subsequent time-delayed swelling of the superabsorbents in the transfer layer has allowed the initial liquid to pass through the transfer layer quickly and easily, but after the initial liquid has passed through, the superabsorbent in the transfer layer swells to close the apertures and prevent an equally rapid transfer back through the apertures upward though the sheet.

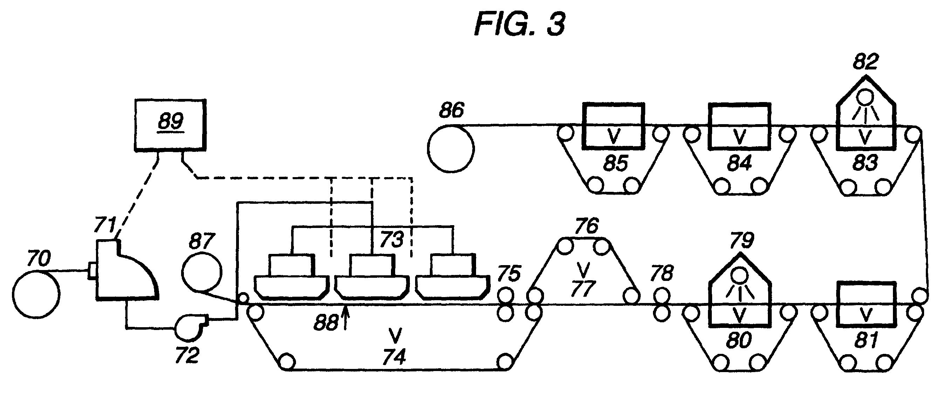

Figure 3 illustrates of a typical air laid machine, incorporating the use of carrier web on the foraminous forming wire to retain the superabsorbent material. Wood pulp (70) is hammermilled (71) and sent via a transport fan (72) to the forming heads (73). A carrier web of wood pulp fibers or other nonwovens consisting of wood pulp and/or synthetic fibers (87) is unwound on top of the forming wire (88). Superabsorbents in the form of particulate or powders (89) are added through the hammermill air stream, directly into the forming heads, or in-between the forming heads to produce a relatively homogenous mixture of superabsorbents and fibers. The fibrous web and superabsorbent mixture is passed through a compaction station (75), the transfer section (76 & 77), an emboss section (78) and into the first spray cabin for latex application (79 & 80). Following this station the web is dried (81) and turned over so that the second side of the web can be bonded (82 & 83). The web is then dried (84 & 85) and wound up into a parent roll (86). The location of the emboss station (78) may optionally be located at other places within the process, as would be evident to one skilled in the art. When using a superabsorbent binder instead of the powders or particles, the superabsorbent binder would be applied at the binder spray stations, 79 & 80, and 82 & 83. When bicomponent fiber bonding or other monocomponent thermoplastic or thermosetting fiber bonding is used in place of or as an adjunct to latex bonding, the bicomponent or moncomponent fibers are added to the forming head (73).

The carrier web (87) may be a wood pulp tissue web made by a number of conventional wet laid tissue making operations. The web may also be a nonwoven web made by the wet laid process in which some short synthetic fibers are added to the wood pulp furnish. Alternatively the web may be a completely synthetic fiber furnish nonwoven web made by any number of nonwovens processes. One function of the web (87) is to provide a particulate barrier to prevent superabsorbent particles or powders from falling out from the interstitial voids of the air laid fibrous web or falling out from the space between the interstitial voids until such time as the particles or powders are bonded into the web. This web must have sufficient porosity to allow a vacuum under the forming wire (74) to aid in the deposition of the fibers from the forming heads (73), but the porosity must be caused by a plurality of apertures small enough so as to still inhibit the passage of the superabsorbent particles or powders while yet allowing the passage of air.

Bonding of the superabsorbent powders or particles within the fibrous structure is most advantageously through the use of latex bonding, during which time the latex also coats part of the surface of the superabsorbent, thereby delaying its reaction time when wetted with a liquid. However, an alternative manner of bonding the superabsorbents in the fibrous structure is the use of bicomponent fibers. Bicomponent fibers are fibers with a different core and sheath. As the sheath melts, it acts as a bonding agent to any materials in direct contact with it. The use of bicomponent fibers is not as desirable as the use of latex bonding, but the use of bicomponents fibers is a viable alternative method for improved retention of superabsorbent powders or particles.

Another embodiment of our invention is the use of superabsorbent binders. When using superabsorbent binders, we do not need the tissue web (89) to retain the superabsorbents. (There may still be some advantage in the use of the tissue web as a density gradient composite for enhanced liquid transfer.) The Superabsorbents are applied at the binder application stations (79/80 and 82/83). When using superabsorbents, there is no need for superabsorbent powders or fibers, but there may be some special cases where the commercial use of SAB's, with SAP's, and or SAF's would still be advantageous.

Once the transfer layer of our composite invention has been formed and bonded, it is then subjected to a hole or aperture making process in which the holes are punched completely through the layer in a vertical or "z" direction. This hole making operation may be done in-line with our web making process, in which case the holes would be generated after the final dryer (85) but before the wind up into a parent roll (86). Or the parent roll (86) may be unwound in some subsequent off-line hole generating operation. Many different processes may be used to punch these holes, and one skilled in the art will readily see that it is not the method or process of how the holes are punched which is important. Rather it is primarily the fact that the holes are punched in sufficient diameter and frequency so as to provide the benefit.

There are many existing hole punching technologies already in the nonwoven industries, such as hydroentangling, aperturing, needle punching. However, the prime design benefit of these technologies is the entangling of fibers in the web, thereby enhancing the strength and integrity of the web. In our invention, any of these technologies may be used with success, but the requirement of strength enhancement is not necessary, and thus utilization of these technologies as the method of hole punching for our invention results in inefficient and wasted energy transfers. In our invention, still other hole making processes such as a needle roll or perfing roll may be used with success, but without the unnecessary addition of fiber entanglement and strength generation.

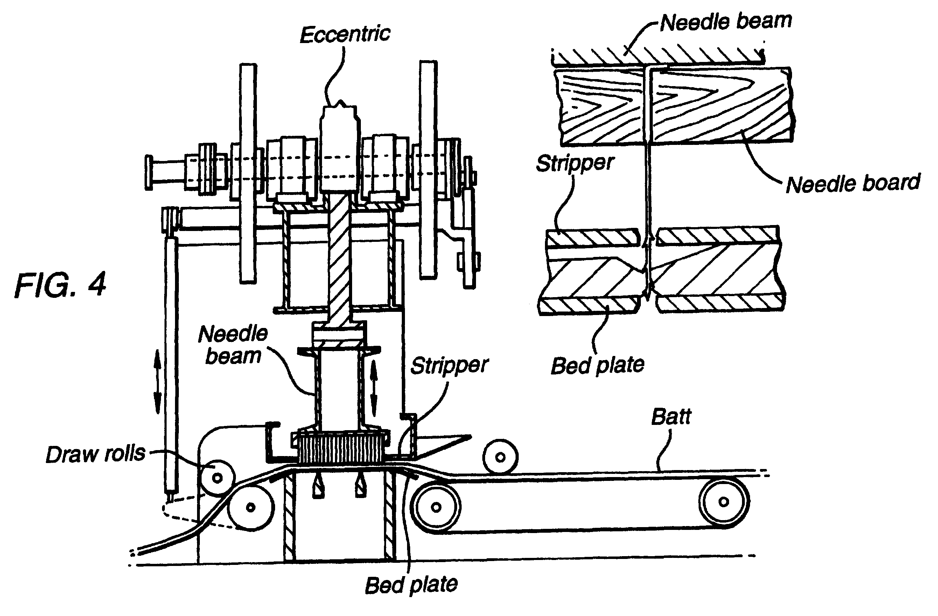

Figure 4 shows a schematic of a needle felting process. The barbed needles are pushed down through the web and then withdrawn. The barbs on the needles are designed to push the fibers down into the web and entangle them during the punching operation, and then freely release the needle as it is drawn out upward. The type of operation is used to enhance the strength of the fibrous web, and/or to create a laminate strength between two adjacent webs that are needled together. Again, in our invention there is no need to create the strength in the transfer layer of our composite, and there is no need to create a laminate strength between two adjacent layers. Thus, in our invention, one would not need a barbed needle, and a smooth needle would suffice. Alternatively, if a needling operation with barbed needles were available, they could still be used for the benefits of our invention by restriction the depth of penetration of the barbed needles so that the needle still penetrated through the entire web, but the barbs did not enter the web.

As an alternative to conventional needle punching without barbed needles, or with controlled depth of needle penetration, a type of needle roll could be used. In this roll, as shown in Figure 5, the smooth needles would be permanently mounted on a cylindrical roll. The transfer layer web of our composite would pass under the roll at a similar surface line speed, causing the needles to enter and completely penetrate the web when they are in a vertical position. As the web progresses and the roll turns, the needles would be withdrawn. Again, although unnecessary, barbed needles could be used.

Still another alternative would be the use of a perforation roll, as is commonly used in tissue and towel making operations to create the tear perfs for easy dispensing. In this case, rather than individual needles, the perf blade would be adjusted with sufficient penetrations and depths and widths of penetration to cause the same performance effects of punched holes. Figure 6 shows a schematic of a perf blade for punching holes in the web of our invention.

As stated earlier, less desirable, but still functionally acceptable methods of adding the holes would also be through the hydraulic needling known in the industry as aperturing and hydroentangling. Aperturing is a less energy intensive form than hydroentanglement, which generally results in larger holes and less entanglement, but both use high pressure water jets to perforate the web and cause fiber entanglement and the generation of holes.

The following examples are illustrative of the present invention. It should be understood that the examples are not intended to limit the invention and that various changes may be made by those skilled in the art without changing the essential characteristics of the invention.

Table 1 shows the composition of various handsheets, and absorbency test data for composite structures of our invention, using these handsheets, both with and without the holes of our invention in the transfer layer of our invention. The hole density for the structure of Table 1 was 11/in², with a needle diameter of 0.45". All holes were punched by hand. The products of our invention are labeled as "perfed" in Table 1.

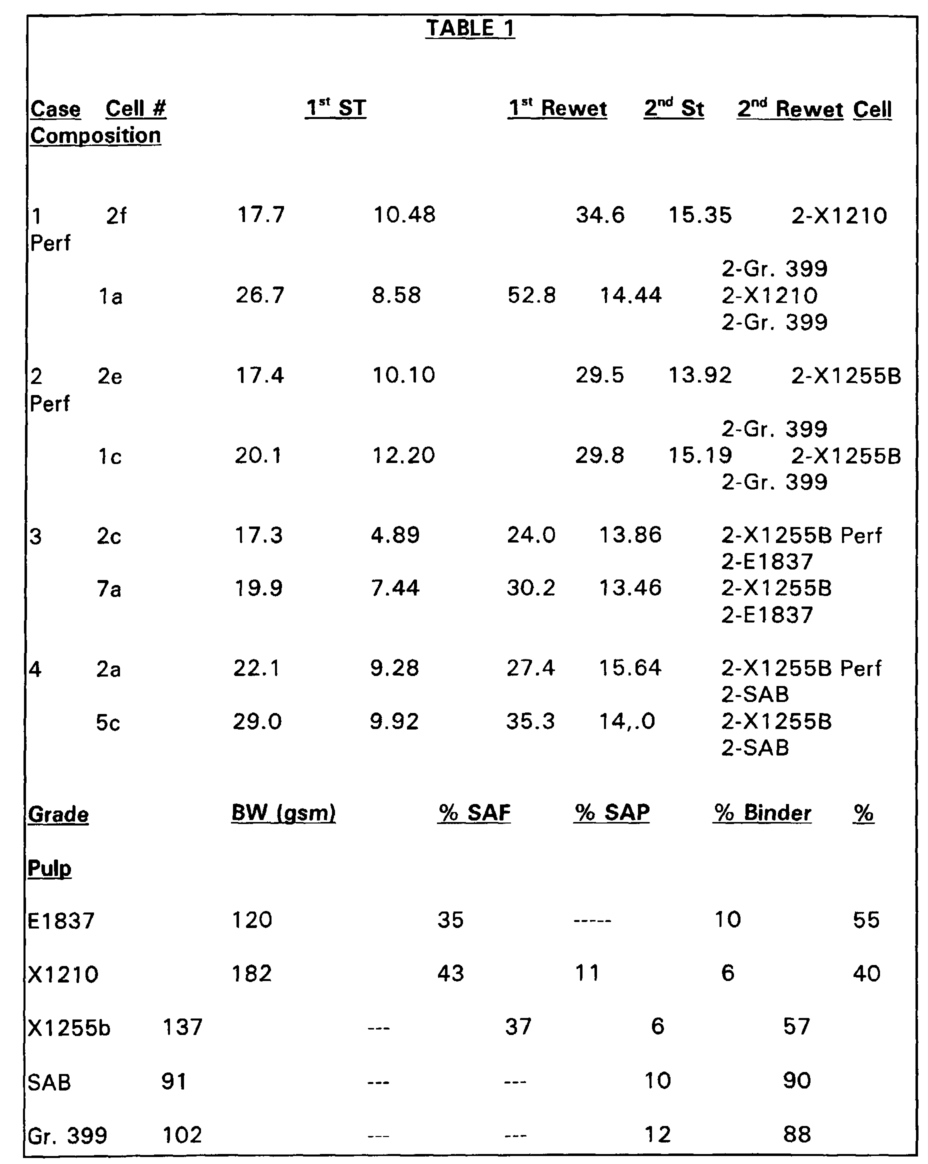

Composite structures consisting of six plies were created for testing. The first ply was a commercially available cover stock consisting of 20.3 gsm thermobonded carded web. The second ply was a commercial available transfer layer consisting of a 40.2 gsm spunlaced web. The third and fourth plies were the superabsorbent containing air laid substrates of our invention, utilized as a specially designed transfer layer. In the paired comparison testing, one cellutilized the holes of our invention and one cell did not. The fifth and sixth plies were the absorbent cores of the composite structure, and they were also superabsorbent containing air laid substrates, with no holes. The superabsorbents used were powders or particlulates (SAP), fibers (SAF), or binder (SAB). In the constructure of the air laid plies used, a typical fluff pulp, such as a southern softwood kraft pulp was used. The fluff pulp may be untreated or treated. A conventional binder used was an ethylene vinyl acetate binder from Air Products and Chemicals, A190; the superabsorbent powder was SMX77 from Stockhausen; the superabsorbent fibers were Oasis 101 from Technical Absorbents; and the superabsorbent binder was PD 8081H from HBFuller. The conventional binder formulation consisted of 10% by solids of the latex binder, 0.1 % Aerosol OT-75 surfactant, 0.1 % by solids of an acid catalyst such as Sodium Bisulfate, and 89.9% water.

The test method involved utilized sample sizes of 4" x 11". The sample was placed under a metal ring of 2.5" O.D. by 1 .875" I.D., weighing 82 grams. An 80 ml insult of 1% saline was poured onto the coverstock surface of the composite structure. A stopwatch was used to measure the time required for the visible liquid to disappear from the surface of the coverstock. This is known as the Strikethrough time. Following the strikethrough, the retaining ring was removed, and the wetted sample was allowed to sit undisturbed for 10 minutes in order to allow the liquid to penetrate the composite and reach a partial equilibrium of distribution. Following this equilibrium time, 10 plies of pre-weighed Whatman #4 filter paper were laid on top of the cover stock. A stack of weights with a face size of 4" x4", weighing 3600 grams was placed on the filter paper, and the system was allowed to stand for 2 minutes. Following this time period, the weights and the filter paper were removed, and the filter paper was reweighed, noting the increase in weight due to the liquid pickup. This increase in weight is known as the Rewet value.

In all cases it can be seen that the 1^{st} strikethrough times of the composites using the holes of our invention are less than those found with the same composites made without the holes of our invention. Faster strikethrough times are desirable, because they represent more rapid movement of liquid away from the insult site, through the cover layer, and into the product composite where it will be contained. This reduced time of strikethrough demonstrates some of the superiority of the product of our invention.

In three out of four cases, all of the test samples using the holes of our invention exhibited less rewet than did their non-hole counterparts. The lesser rewets in the products of our invention may be readily attributed to the fact that the superabsorbents in the transfer layers of our product had swelled shut the holes after the first insult, preventing significant rewet. This again demonstrates some of the superiority of the product of our invention.

Following the measurement of rewet, a waiting period of 2 minutes was allowed to elapse, and then, the products in Table 1 were then re-insulted for further testing. One might logically expect that the second strikethrough values and second rewet values of the products of our invention might not show any benefit in that the holes have swelled shut. Surprisingly we have found that the second strikethrough values of the products of our invention continue to show superiority in all cases, while the second rewets show mixed results. In all cases, the 2^{nd} strikethrough times in the products of our invention were less than the strikethrough times of the identical products that did not have z-direction apertures in the transfer layer. The exact mechanism of why our invention continues to show superiority is not fully understood.

Table 2 shows the composition of some web components made on a conventional commercial air laid line, along with the test data for a composite structure of two plies. In Table 2, the raw materials used in the basesheets were a southern softwood kraft fluff wood pulp, a conventional ethylene vinyl acetate binder system, and SanWet IM 7050P SAP from BASF. The apertures in the substrates of Table 2 were produced by conventional needle punching. Aperture frequency was 14/in², and the needle and aperture diameter was 0.037". In Table 2, the apertures are listed as "perfs". In Table 2, however, the composite sample variables of the commercial cover sheet and transfer layer were removed. Furthermore, only two plies of the air laid substrates were used, one acting as the transfer layer both with and without the apertures, and one acting as the absorbent core of the composite. To accommodate the lesser structure of the composites in Table 2, only 50 ml of insult saline was used, and only one insult test was performed.

The data in Table 2 also clearly shows the superiority of the composite structure of our invention. The average strikethrough times of the products of our invention were 18% less than the control values.

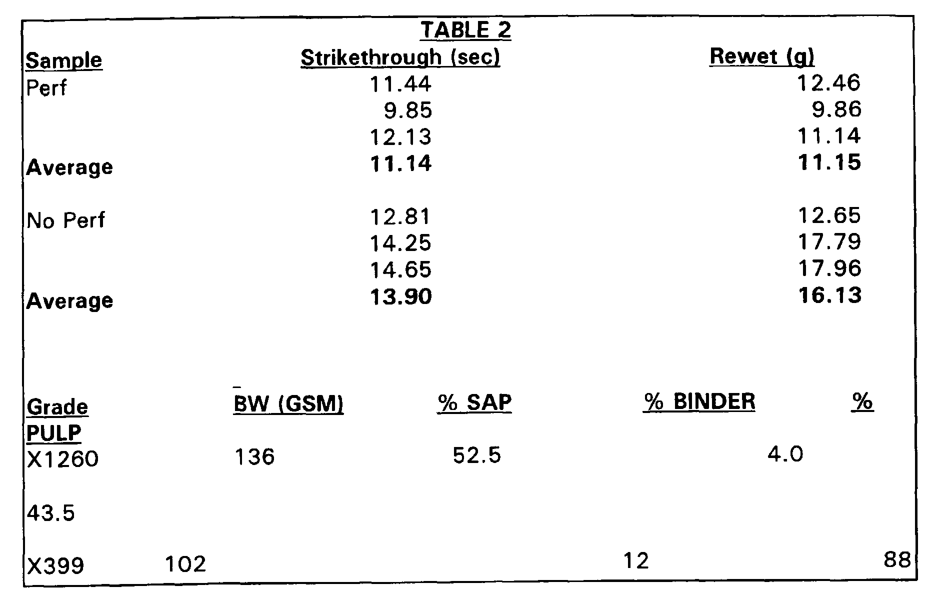

## Claims

1. A multilayered absorbent composite comprising a cover sheet, one or more transfer layers, one or more absorbent core layers and a barrier back layer characterised in that the transfer layer or layers have Z-direction apertures penetrating through the entire layer wherein the aperture frequency is from 5 to 50 apertures per square inch and wherein each aperture has a diameter of about 0.015 to 0.10 inches, further characterised in that a superabsorbent composition is distributed homogeneously throughout at least one of the transfer layers.

2. A composite according to claim 1 wherein the Z-direction apertures in the transfer layer were made by flat bed needle punching, per roll punching, needle roll punching, hydroentangling or aperturing.

3. A composite according to claim 1 or 2 wherein the transfer layer is comprised of cellulosic fibers.

4. A composite according to any preceding claim wherein the transfer layer includes synthetic fibers selected from the group consisting of regenerated cellulose, polyolefins, polyethylenes, polypropylenes, polyesters, polyimides, polyamides, inorganic polymers, silica polymers and carbon fibers.

5. A composite according to any preceding claim wherein the absorbent core layer or layers and/or the superabsorbent composition of the transfer layer of layers comprises organic polymers crosslinked with one or more of the following crosslinkers; zirconium having a valency of plus four, sodium salt, potassium salt, lithium salt, aluminium, ammonium salt, alkylene carbonate, dimethylaminoethyl methacrylate, long chain epoxy ether, ethylene glycol, diallyl ester, ethylene glycol dialkyl ester, ethylene glycol diglycidyl ether, laurylmethacrylate, methylene bisacrylamide, polyethylene glycol diacrylate/methacrylate, polyethylene glycol-mono acrylate/methacrylate, polyethylenimine, tetraallyloxyethane, tetraalkyloxyethane, tetramethylsuccinonitrile, triallylamine, triethylene glycol dimethyl acrylate, trimethylolpropane, and trimethacrylate.

6. A composite according to any preceding claim wherein the organic polymers in the absorbent core layer are selected from the following: carboxymethylcellulose, hydrolyzed starch polyacrylate graft copolymer, hydrolyzed starch polyacrylonitrile graft copolymer; polyacrylamide, polyacrylic acid, polyisobutylene maleic anhydride copolymer.

7. A composite according to any preceding claim wherein the fibers in the transfer layer or layers are bonded together by latex bonding, bicomponent fiber bonding, monocomponent thermoplastic bonding or with thermosetting bonding fibers.

8. A composition according to claim 7 wherein the bicomponent binder fiber is selected from the group consisting of polyester, polypropylene, and polyethylene; the monocomponent thermoplastic binder is selected from the group consisting of polyethylene and polypropylene; and the thermosetting binder fibers are selected from the group consisting of polyethylene, polypropylene, and polyester.

9. A composite according to any preceding claim wherein the organic polymers comprising the superabsorbent in the transfer layer or layers is selected from the following: carboxymethylcellulose, hydrolysed starch, polyacrylonitrile graft copolymer, polyacrylamide, polyacrylic acid, polyisobutylene maleic anhydride copolymer.

10. A composite according to any preceding claim wherein the superabsorbent is selected from the group consisting of polyacrylate polymers and their sodium, lithium or potassium salts, and a crosslinker.

11. A composite according to any preceding claim wherein the superabsorbent comprised of a crosslinker and a salt is selected from the sodium, potassium and lithium salt of methyl acrylate, ethyl acrylate, propyl acrylate, hexyl acrylate, butyl methacrylates, hexyl methacrylates, octyl methacrylate, decyl methacrylate, 2-hydroxyethyl acrylate, hydroxymethyl acrylate, 3-hydroxyl-propyl acrylate and 4-hydroxybutyl acrylate.
